Europäisches Patentamt

European Patent Office

Office européen des brevets

(11) Publication number: **0 408 782 A1**

# EUROPEAN PATENT APPLICATION

(21) Application number: 89113183.1

(22) Date of filing: 18.07.89

(51) Int. Cl.⁵: **A61K 31/40, A61K 31/495**

(43) Date of publication of application:
23.01.91 Bulletin 91/04

(84) Designated Contracting States:
**BE CH DE FR GB IT LI NL**

(71) Applicant: **NAUCHNOISSLEDOVATELSKI CHIMIKOPHARMATZEVTICHEN INSTITUT**
**3, Boul.Kl.Ohridski**
**BG-1156 Sofia(BG)**

(72) Inventor: **Tzoneva-Tyutyulkova, Nadejda**
**11, Graf Ignatiev Street**
**Sofia(BG)**
Inventor: **Kostov Nikolov, Rumen**
**12b, Rozova Dolina Street**
**Sofia(BG)**
Inventor: **Petrova Nikolova, Milka**
**3, R.Daskalov-Square**
**Sofia(BG)**
Inventor: **Hinova Andonova, Violeta**
**Komplex Tolstoy, B1.68-B**
**Sofia(BG)**
Inventor: **Georgiev Georgiev, Atanas**

**1, Lakatitza Street**
**Sofia(BG)**
Inventor: **Assenov Ninov, Kiril**
**18, Sultan-Tepe Street**
**Sofia(BG)**
Inventor: **Petrov Daskalov, Hristo**
**Boul. Lenin, Block 1**
**Sofia(BG)**
Inventor: **Nissim Nissimov, Jossif**
**37, Sofronii Street**
**Sofia(BG)**
Inventor: **Ivanov Hadjiev, Dimiter**
**Komplex Lyulin, Block 21-A**
**Sofia(BG)**
Inventor: **Todorova Yancheva, Stefka**
**Komplex Lyulin, Block 419-4**
**Sofia(BG)**

(74) Representative: **von Füner, Alexander, Dr. et al**
**Patentanwälte v. Füner, Ebbinghaus, Finck**
**Mariahilfplatz 2 & 3; 3**
**D-8000 München 90(DE)**

(54) Antihypoxic agent.

(57) The antihypoxic drug of a pharmaceutical composition comprising a combination of 1-cinnamyl-4-(di-p-fluoro-benzhydryl)-piperazine (flunarizine) and 2-oxo-1-pyrrolidineacetamide (piracetam) is described.

The pharmacological data showed that the combined administration of flunarizine and piracetam in two models of brain hypoxia ( hypobaric and decapitation) lead to mutual potentiation of their antihypoxic effect.

Toxicological studies show a strong decrease of the acute toxicity of the combination in comparison with the more toxic component flunarizine.

The advantages of the pharmaceutical combination is enhanced cerebro-protective effect and significant reduction of the acute toxicity of flunarizine which might reduce its side effects of extrapyramidal origin.

## ANTIHYPOXIC AGENT

This invention refers to a pharmaceutical agent for the treatment of diseases related to hypoxic and ischaemic disturbances of cerebral functions, such as: acute and chronic impairment of cerebral circulation, post-insult and post-traumatic states, cerebral atherosclerosis, migraine, senile dementia, etc.

1-cinnamyl-4-(di-p-fluoro-benzhydryl-piperazine is well known under the generic name flunarizine. This pharmaceutical agent is applied in clinical practice as a central and peripheral vasodilator, acting as Ca$^{2+}$ entry blocker (1).

Disadvantages of flunarizine are its side effects of extrapyramidal origin, observed in patients (2-5).

Well-known is also piracetam, (10) which is 2-oxo-1-pyrrolidineacetamide. It is widely used in clinical practice for the treatment of a number of cerebral disturbances, such as cerebral trauma, cerebro-vascular disturbances, senile dementia, as well as diseases connected with brain hypoxia (6,7). Piracetam does not possess a vasodilator effect.

Well known is an antihypoxic agent containing 2-oxo-1-pyrrolidineacetamide (piracetam) and 1-cinnamyl-4-benzhydrylpiperazine (cinnarizine), which is applied in medical practice for the treatment of disturbances of cerebral circulation and metabolism, especially in states of hypoxia under the name phezam. It possesses a relatively weak anti-ischaemic effect.

It is the objective of this invention to create a pharmaceutical preparation, possessing vasodilator, antihypoxic and anti-ischaemic effects, lacking side effects of extrapyramidal origin.

This objective is solved through the development of a pharmaceutical preparation, containing 1-cinnamyl-4-(di-p-fluoro-benzhydryl)-piperazine (flunarizine) and 2-oxo-1-pyrrolidineacetamide (piracetam) in quantity ratios as follows: from 5 mg to 15 mg flunarizine and from 100 mg to 800 mg piracetam.

The combination of the active ingredients piracetam and flunarizine is suitable for the production of pharmaceutical compositions and dosage unit form which can contain the active ingredients or their pharmaceutically tolerated salts, mixed with the conventional excipients, as shown in the examples.

The pharmaceutical preparation of this invention can be applied orally or in some cases parentherally.

The advantages of the agent as per this invention are as follows:
- an enhanced anti-hypoxic, anti-ischaemic and cerebro-protective effect;
- a significant reduction of the side effects of extrapyramidal origin.

The following examples illustrate this invention:

EXAMPLE 1: Pharmacological study: Cerebro-protective anti-hypoxic effect using the method "hypobaric hypoxia" in mice.

The experiments are carried out on white mice line H (18-22 g) in groups of 8 animals. The hypobaric hypoxia was provoked by means of the method of Nakanishi et al.(8) - The mice were placed in a decompression chamber, in which the atmospheric pressure was sharply reduced to 210 mmHg (corresponding to an altitude of about 9500 m). The survival time (in seconds) from the beginning of the hypoxia till the death of the animals was measured. The results were calculated as a percentage towards the control group. Flunarizine and piracetam were administered intraperitoneally separately or in combination 1 hour before the hypoxia (table 1).

Table 1

| Effect or flunarizine in combination with piracetam in a model of hypobaric hypoxia in mice. | | |
|---|---|---|
| Pharmeceutical agent or pharmaceutical compositor | Dosage (mg/kg) | Increase of the resistancy towards hypoxia (% towards the control group) |
| Flunarizine | 10 | 11,7 |
| Piracetam | 1600 | 22,8 |
| Combination: | | |
| Flunarizine + Piracetam (in a weight ratio 1:160) | 10 + 1600 | 42,1 |
| Phezam | 1600 | 42,9 |

2

It is found out that during the simultaneous administration of flunarizine and piracetam, a mutual potentiation of their antihypoxic effects is observed, which is expressed in an increased cerebro-protective effect. The pharmacological investigation shows that the supraadditive antihypoxic effect is 7,6% towards the control group.

Example 2. Pharmacological study of the cerebro-protective anti-ischaemic effect using the method of "complete ischaemia by decapitation" in mice.

The experiments are carried out on white mice line H (18-22g) in groups of 8 animals. Complete ishaemia was caused by means of a decapitation of the animals using the method of Hollowach-Thurston et al.(9). The duration of the respiratory movements of the isolated head (in seconds) was measured. The results are calculated as a percentage towards the control group. Flunarizine and piracetam were administered intraperitoneally separately or in combination 1 hour before the hypoxia (table 2).

Table 2

| Effect of flunarizine in combination with piracetam in a model of complete ischaemia caused by means of a decapitation of mice. | | |
|---|---|---|
| Pharmaceutical agent or pharmaceutical composition | Dosage (mg/kg) | Increase of the resistance towards ischaemia (% towards the control group) |
| Flunarizine<br>Piracetam | 10<br>1600 | 42,8<br>27,9 |
| Combination: | | |
| Flunarizine + Piracetam (in a weight ratio 1:160)<br>Phezam | 10 + 1600<br>1600 | 86,8<br>36,9 |

It is found out that during the simultaneous administration of flunarizine and piracetam, a mutual potentiation of their antiischaemic effects occurs. The supraadditive antiischaemic effect is 15,1% towards the control group (Table 2).

Example 3. Toxicological investigations

The toxicological studies of the acute toxicity of the agent as per this invention show that during oral administration the $LD_{50}$ of the combination flunarizine + piracetam in a weight ratio 1:160 is 5000 mg/kg and during intraperitoneal - 5000 mg/kg, but $LD_{50}$ of flunarizine is 232 mg/kg during oral and 204 mg/kg during intraperitoneal administration. The data show that the acute toxicity of the combination flunarizine + piracetam is decreased 21 times at oral and 25 times at intraperitoneal administration (Table 3) compared with the acute toxicity of the more toxic component - flunarizine. The combination of flunarizine with piracetam places said pharmaceutical agent of the invention in the class of the practically non-toxic pharmaceuticals.

EP 0 408 782 A1

Table 3

| Acute toxicity of flunarizine and piracetam and the combination flunarizine + piracetam in mice. | | |
|---|---|---|
| Pharmaceutical agent or pharmaceutical combination | Mode of administration | $LD_{50}$ mg/kg |
| Flunarizine<br><br>Piracetam | orally<br>intraperitoneally<br>orally<br>intraperitoneally | 232/182 + 295/<br>204/158 + 264/<br>8000<br>8000 |
| Combination: | | |
| Flunarizine + Piracetam (in weight ratio 1:160)<br><br>Phezam | orally<br>intraperitoneally<br>orally | 5000<br>5000<br>3000 |

| Example 4. Contents of one capsule in g: | |
|---|---|
| Piracetam | 0,4000 |
| Flunarizine | 0,0050 |
| Glycine | 0,0150 |
| Microcrystalline cellulose (Avicel) | 0,050 |
| Aerosil 200 | 0,015 |
| Wheat starch | 0,0305 |
| Talc | 0,0100 |
| Magnesium Stearate | 0,0080 |

BIBLIOGRAPHY

1. Holmes, B.,N.R.Brodgen, R.C.Heel, T.M. Speight, G.S.Avery Flunarizine - A Review of its pharmacodynamic and pharmacokinetic properties and therapeutic use. Drugs, 1984, 27, 6-44.

2. Chouza C., J.L.Caamano, R.Aljanati, A.Scaramelli, O.De Medin, S.Romero. Parkinsonism, tardive dyskinesia, akathisia and depression induced by Flunarizine. Lancet, 1986, 1, 1303-1304.

3. Benvenuti, F., A.Baroni, S.Bandinelli, P.Sommazzi, R.Corradetti, T.Pantalbo. Side effects of Flunarizine. Lancet, 1986, II, 464.

4. Meyboom, .H.B., M.D.Ferrari, B.P.Dieleman. Parkinsonism, tardive dyskinesia, akathisia and depression induced by Flunarizine. Lancet, 1986, II, 292.

5. D'Alessandro,R., G.Benassi, G.Morganti. Side-effects of Flunarizine. Lancet, 1986, II,463.

6. Stegink, A.I. The clinical use of Piracetam a new nootropic drug. The treatment of symptoms of senile involution. arzneim. Forsch., 1972,22,975-977.

7. Hadjiev, D., .Yancheva, R.Tsikalova. Pyramen (Piracetam). Rheoevcephalographic and electroencephalographic studies on its effects in latent insufficiency of cerebral circulation, MBI, 1985, 5,12-15.

8. Nakanishi,M., H.Yasuda, T.Tsumagari. Life Sci.,1973, 13, 467-477.

9. Hollowach- Thuston, J., R.E.Hauart, E.M.Jones. Pediat. Res.,1974,8,238-243.

10. Georgiev A.,K.Konstantinova, H.Daskalov et al. Czechoslovak patent 242951 (1986).

**Claims**

An antihypoxic drug as a pharmaceutical composition comprising a combination of the compounds 1-cinnamyl-4-(di-p-fluoro-benzhydryl)-piperazine and 2-oxo-1-pyrrolidineacetamide, which combination is in

4

dosage unit form and each dosage unit containing 1-cinnamyl-4-(di-p-fluoro-benzhydryl)-piperazine from 1 mg to 15 mg and 2-oxo-1-pyrrolidineacetamide from 100 mg to 800 mg.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOMED. BIOCHIM. ACTA, vol. 48, nos. 2/3, 1989, pages 243-246; C. WUSTMANN et al.: "New aspects in mechanisms of antihypoxic piracetam action", & INTERNATIONAL SYMPOSIUM ON HYPOXIA, East Berlin, 12th-14th September 1988 * Page 243, summary * | 1 | A 61 K  31/40 A 61 K  31/495 |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 15-03-1990 | LEHERTE C.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document